# EUROPEAN PATENT APPLICATION

(11) **EP 1 821 103 A1**
(43) Date of publication of application: **22.08.2007**
(21) Application number: 06003238.0
(22) Date of filing: 17.02.2006
(51) Int. Cl.: G01N 33/68

(54) **Methods for the identification of gama-secretase modulators**

(71) Applicant: CELLZOME AG, 69117 Heidelberg (DE)
(72) Inventor: Drewes, Gerard, 69121 Heidelberg (DE); Stein, Martin, 68307 Mannheim (DE)
(74) Representative: Huhn, Michael

(57) **Abstract**

The invention relates to a method for identifying a gamma-secretase modulator, comprising the following steps:
a) identifying a Pen-2 interacting compound, and
b) determining whether the Pen-2 interacting compound identified in step a) is capable of modulating gamma-secretase activity.

## Description

The present invention relates to methods for the identification of gamma-secretase modulators as well as to pharmaceutical compositions comprising such modulators.

Alzheimer's disease is a chronic condition that affects millions of individuals worldwide.

The brains of sufferers from Alzheimer's disease show a characteristic pathology of prominent neuropathologic lesions, such as the initially intracellular neurofibrillary tangles (NFTs), and the extracellular amyloid-rich senile plaques. These lesions are associated with massive loss of populations of CNS neurons and their progression accompanies the clinical dementia associated with AD. The major component of amyloid plaques are the amyloid beta (A-beta, Abeta or Aβ) peptides of various lengths. A variant thereof, which is the Aβ1-42-peptide (A beta-42), is the major causative agent for amyloid formation. Another variant is the Aβ1-40-peptide (Abeta-40). Amyloid beta is the proteolytic product of a precursor protein, beta amyloid precursor protein (beta-APP or APP). APP is a type-I trans-membrane protein which is sequentially cleaved by several different membrane-associated proteases. The first cleavage of APP occurs by one of two proteases, alpha-secretase or beta-secretase. Alpha-secretase is a metalloprotease whose activity is most likely to be provided by one or a combination of the proteins ADAM-10 and ADAM-17. Cleavage by alpha-secretase precludes formation of amyloid peptides and is thus referred to as non-amyloidogenic. In contrast, cleavage of APP by beta-secretase is a prerequisite for subsequent formation of amyloid peptides. This secretase, also called BACE1 (beta-site APP-cleaving enzyme), is a type-I transmembrane protein containing an aspartyl protease activity (described in detail below).

The beta-secretase (BACE) activity cleaves APP in the ectodomain, resulting in shedding of secreted, soluble APPb, and in a 99-residue C-terminal transmembrane fragment (APP-C99). Vassar et al., 1999, (Science 286, 735-741) cloned a transmembrane aspartic protease that had the characteristics of the postulated beta-secretase of APP, which they termed BACE1. Brain and primary cortical cultures from BACE1 knockout mice showed no detectable beta-secretase activity, and primary cortical cultures from BACE knockout mice produced much less amyloid-beta from APP. This suggests that BACE1, rather than its paralogue BACE2, is the main beta-secretase for APP. BACE1 is a protein of 501 amino acids (aa) containing a 21-aa signal peptide followed by a prosequence domain spanning aa 22 to 45. There are alternatively spliced forms, BACE-1-457 and BACE-1-476. The extracellular domain of the mature protein is followed by one predicted transmembrane domain and a short cytosolic C-terminal tail of 24 aa. BACE1 is predicted to be a type 1 transmembrane protein with the active site on the extracellular side of the membrane, where beta-secretase cleaves APP and possible other yet unidentified substrates. Although BACE1 is clearly a key enzyme required for the processing of APP into A-beta, recent evidence suggests additional potential substrates and functions of BACE1 (Li and Südhof, 2004, J. Biol. Chem. 279, 10542-10550). To date, no BACE1 interacting proteins with regulatory or modulatory functions have been described.

The APP fragment generated by BACE1 cleavage, APP-C99, is a substrate for the gamma-secretase activity, which cleaves APP-C99 within the plane of the membrane into an A-beta peptide (such as the amyloidogenic Aβ1-42 peptide), and into a C-terminal fragment termed APP intracellular domain (AICD) (Annu Rev Cell Dev Biol 19, 25-51).

The gamma-secretase activity resides within a multiprotein complex containing at least four components: the presenilin (PS) heterodimer, nicastrin, aph-1 and Pen-2. The PS heterodimer consists of the amino- and carboxyterminal PS fragments generated by endoproteolysis of the precursor protein. The two aspartates of the catalytic site are at the interface of this heterodimer. It has recently been suggested that nicatrin serves as gamma-secretase-substrate receptor. The functions of the other members of gamma-secretase are unknown, but they are all required for activity (Steiner, 2004. Curr. Alzheimer Research 1(3): 175-181).

Despite recent progress in delineating molecular events underlying the etiology of Alzheimer's disease, no disease-modifying therapies have been developed so far. To this end, the industry has struggled to identify suitable lead compounds for inhibition of BACE1. Moreover, it has been recognized that a growing number of alternative substrates of gamma-secretase exist, most notably the Notch protein. Consequently, inhibition of gamma-secretase is likely to cause mechanism-based side effects. Current top drugs (e.g. Aricept®/donepezil) attempt to achieve a temporary improvement of cognitive functions by inhibiting acetylcholinesterase, which results in increased levels of the neurotransmitter acetylcholine in the brain. These therapies are not suitable for later stages of the disease, they do not treat the underlying disease pathology, and they do not halt disease progression.

Various strategies have been proposed for targeting gamma-secretase in Alzheimer's disease, ranging from targeting the catalytic site directly, developing substrate-specific inhibitors and modulators of gamma-secretase activity (Marjaux et al., 2004. Drug Discovery Today: Therapeutic Strategies, Volume 1, 1-6).

WO 04/007544 describes gamma-secretase protein complexes and methods for screening for a molecule that binds to said complexes.

Accordingly, a variety of compounds were described that have secretases as targets (Larner, 2004. Secretases as therapeutics targets in Alzheimer's disease: patents 2000 - 2004. Expert Opin. Ther. Patents 14, 1403-1420.)

Epidemiological studies revealed a reduced prevalence of Alzheimer's disease among users of non-steroidal anti-inflammatory drugs (NSAIDs) and therefore it was suggested to use NSAIDs as protective factors for Alzheimer's disease (McGeer et al., 1996. Arthritis and anti-inflammatory agents as possible protective factors for Alzheimer's disease: a review of 17 epidemiologic studies. Neurology 47, 425-432). The principal pharmacological targets of NSAID drugs are the cyclooxygenase (cox) enzymes and it was originally expected that the protective activity in Alzheimer's would also be mediated by these targets.

Subsequently it was demonstrated that a subset of NSAIDs lowers the production of amyloidogenic Abeta42 peptides independently of Cox activity (Weggen et al., 2001. Nature 414, 212-216). In this study the authors used cell lines deficient in both Cox-1 and Cox-2 enzymes, but they could still demonstrate the Abeta42 lowering effect of a subset of NSAIDs including sulindac sulfide. However, the authors were not able to delineate the molecular mode of action and suggested two potential mechanisms. One mechanism invokes the stimulation of an exopeptidase that degrades Abeta42 peptides. The other would be a modulating influence on the activity of gamma-secretase itself, the enzyme responsible for the generation of Abeta42 peptides.

Eriksen and colleagues further expanded these studies by testing 20 commonly used NSAIDs for their ability to lower Abeta42 peptide levels (Eriksen et al., 2003. J. Clin. Investigation 112, 440-449). It was demonstrated that eight FDA-approved NSAIDs lower Abeta42 peptide levels in vivo and that this effect in mice occurs at drug levels achievable in humans. In addition, flurbiprofen and its enantiomers lowered Abeta42 peptide levels in broken cell gamma-secretase assays.

Beher et al, 2004, further investigated the mode of action of NSAIDs on gamma-secretase and postulated a novel allosteric binding site on gamma-secretase. This novel site is different from the binding site for transition-state analogue gamma-secretase inhibitors. The authors conclude that it still remains to be elucidated which of the polypeptides constituting the gamma-secretase complex is the molecular entity targeted by NSAID derivatives (Beher et al., 2004. J. Biol. Chem. 279, 43419-43426).

An alternative mechanism of action for NSAIDs was recently proposed by Sastre and colleagues (Sastre et al., 2006. Proc. Nat. Acad. Sci. 103, 443-448). Sastre et al. suggest that the protective effect of NSAIDs in Alzheimer's disease involves the activation of the peroxisome proliferator activated receptor-γ (PPARγ) and a decrease in BACE 1 gene transcription. PPARγ is a nuclear trancriptional regulator that can act as a repressor of transcription. Certain NSAIDs (e.g. Ibuprofen, Indomethacin and Naproxen) can bind to and activate PPARγ (PPARγ agonists) which leads in turn to repression of the BACE1 promoter.

The identification of the protein target, preferably a defined binding site, for NSAIDs on gamma-secretase would be of great value for drug discovery, because this would allow the design of target-based screens for new gamma-secretase modulators. Currently, screening for the identification of novel Abeta42-lowering agents is usually performed in cell-based phenotypic assays which have a number of limitations, e.g. an ELISA-based readout for Abeta peptides in cell culture medium. In addition, a target-based biochemical assay would be useful for further optimization (e.g. improved potency) for the NSAIDs known to lower Abeta42. Furthermore, the protein target could be used for the generation of crystal and co-crystal structures providing the basis for structure-based drug design.

Thus, there is an urgent need for the identification of novel targets enabling novel molecular strategies for the treatment of Alzheimer's disease. In addition, there is a strong unmet medical need for novel therapeutic compounds modifying the aforementioned molecular processes by targeting said novel targets.

In a first aspect, the invention relates to a method for identifying a gamma-secretase modulator, comprising the following steps:
a) identifying a Pen-2 interacting compound, and
b) determining whether the Pen-2 interacting compound identified in step a) is capable of modulating gamma-secretase activity.

In the context of the present invention, it has been surprisingly found that a binding site for NSAIDs is found on Pen-2. This binding site is shown in SEQ ID:2. This emphasizes the importance of Pen-2 as a target for compounds useful in the therapy of Azheimer's disease and should enable the use of Pen-2 in screening methods for the identification of compounds which by binding to Pen-2 modulate gamma-secretase activity. As explained above, targeting intracellular sites of Abeta generation is a very attractive Abeta-lowering strategy, as recent evidence suggests that, differing from the cleavage mechanism of other gamma-secretase substrates such as Notch, proteolytic processing of APP is independent of cell surface regulation by extracellular ligands and may instead be controlled intracellularly (Kvotchev and Sudhof, 2004. J. Biol. Chem. 279,47101-47108).

In the context of the present invention, a "Pen-2 interacting compound" is a molecule or a molecular complex which binds at least temporarily to Pen-2.

Pen-2 was first identified through a genetic screen in C. elegans as an enhancer of PS activity. The human Pen-2 gene encodes a 101 amino acid polypeptide that has a hairpin-like topology with the amino and carboxy terminus exposed to the lumen. During the process of gamma-secretase assembly Pen-2 is incorporated into the PS-Nicastrin-Aph 1 trimeric intermediate. It has been reported that Pen-2 provokes the generation of PS fragments and binds to PS (Watanabe et al., 2005. J. Biol. Chem. 280, 41967-41975).

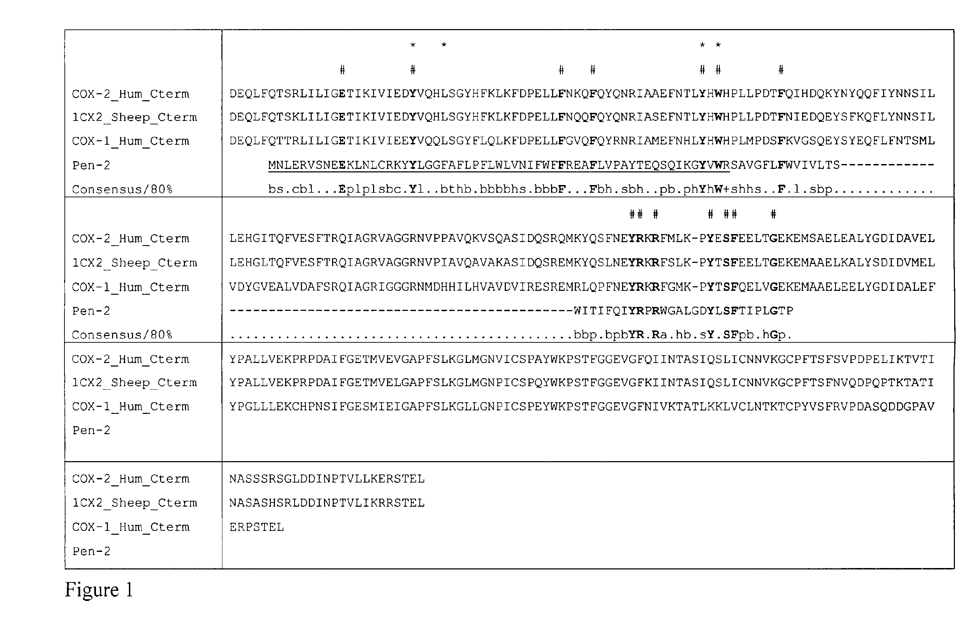

According to the present invention, the expression "Pen-2" does not only mean the protein as shown in Fig. 1, but also a functionally active derivative thereof, or a functionally active fragment thereof, or a homologue thereof, or a variant encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1 % SDS for 1.5 hours at 60°C.

The same applies also to all other proteins named in the present invention. Therefore, a name of given protein or nucleic acid does not only refer to the protein or nucleic acid as depicted in the sequence listing, but also to its functionally active derivative, or to a functionally active fragment thereof, or a homologue thereof, or a variant encoded by a nucleic acid that hybridizes to the nucleic acid encoding said protein under low stringency conditions, preferably under the conditions as mentioned above.

Consequently, a "functionally active derivative" of Pen-2 means in this case a derivate which is also part of the gamma-secretase complex and exerts a comparable influence on the gamma-secretase activity.

In the case of other proteins, the term "functionally active" as used herein refers to a polypeptide, namely a fragment or derivative, having structural, regulatory, or biochemical functions of the protein according to the embodiment of which this polypeptide, namely fragment or derivative, is related to.

According to the present invention, the term "activity" as used herein, refers to the function of a molecule in its broadest sense. It generally includes, but is not limited to, biological, biochemical, physical or chemical functions of the molecule. It includes for example the enzymatic activity, the ability to interact with other molecules and ability to activate, facilitate, stabilize, inhibit, suppress or destabilize the function of other molecules, stability, ability to localize to certain subcellular locations. Where applicable, said term also relates to the function of a protein complex in its broadest sense.

According to the present invention, the terms "derivatives" or "analogs of component proteins" or "variants" as used herein preferably include, but are not limited, to molecules comprising regions that are substantially homologous to the component proteins, in various embodiments, by at least 30%, 40%, 50%, 60%, 70%, 80%, 90%, 95% or 99% identity over an amino acid sequence of identical size or when compared to an aligned sequence in which the alignment is done by a computer homology program known in the art, or whose encoding nucleic acid is capable of hybridizing to a sequence encoding the component protein under stringent, moderately stringent, or nonstringent conditions. It means a protein which is the outcome of a modification of the naturally occurring protein, by amino acid substitutions, deletions and additions, respectively, which derivatives still exhibit the biological function of the naturally occurring protein although not necessarily to the same degree. The biological function of such proteins can e.g. be examined by suitable available in vitro assays as provided in the invention.

The term "fragment" as used herein refers to a polypeptide of at least 10, 20, 30, 40 or 50 amino acids of the component protein according to the embodiment. In specific embodiments, such fragments are not larger than 35, 50 or 101 amino acids. In another specific embodiment, such a fragment consists of the N-terminal 59 amino acids comprising the NSAID binding site.

The term "gene" as used herein refers to a nucleic acid comprising an open reading frame encoding a polypeptide of, if not stated otherwise, the present invention, including both exon and optionally intron sequences.

The terms " homologue" or "homologous gene products" as used herein mean a protein in another species, preferably mammals, which performs the same biological function as the a protein component of the complex further described herein. Such homologues are also termed "orthologous gene products". The algorithm for the detection of orthologue gene pairs from humans and mammalians or other species uses the whole genome of these organisms. First, pairwise best hits are retrieved, using a full Smith-Waterman alignment of predicted proteins. To further improve reliability, these pairs are clustered with pairwise best hits involving Drosophila melanogaster and C. elegans proteins. Such analysis is given, e.g., in Nature, 2001, 409:860-921. The homologues of the proteins according to the invention can either be isolated based on the sequence homology of the genes encoding the proteins provided herein to the genes of other species by cloning the respective gene applying conventional technology and expressing the protein from such gene, or by isolating proteins of the other species by isolating the analogous complex according to the methods provided herein or to other suitable methods commonly known in the art.

In a preferred embodiment of the present invention, the "PEN-2 interacting compound" is a compound which binds to Pen-2, preferably to the Pen-2 binding site as defined in SEQ ID NO:2.

Examples of such Pen-2 interacting compounds are binding proteins or binding peptides binding to Pen-2. Further examples are low molecular weight molecules (LMWs), aptamers, and peptidomimetics. Preferably, the Pen-2 interacting compound belongs to the group consisting of synthetic compounds, or organic synthetic drugs, peptides or aptamers, or natural compounds.

So-called "low molecular weight molecules" (in the following called "LMWs") are molecules which are not proteins, peptides, antibodies or nucleic acids, and which exhibit a molecular weight of less than 1000 Da, preferably less than 750 Da, most preferably less than 500 Da. Such LMWs may be identified in high-throughput procedures starting from libraries. Such methods are known in the art and are discussed in detail below.

The term "binding protein" or "binding peptide" refers to a class of proteins or peptides which bind to Pen-2, and includes, without limitation, polyclonal or monoclonal antibodies, antibody fragments and protein scaffolds directed against Pen-2.

These binding proteins can either be synthesized outside the cell and then introduced into the cell by methods known to the person skilled in the art or they can be expressed intracellularly, again by methods known to the person skilled in the art.

Methods for the production of proteins outside of the cell are widely known in the art, also protocols for the introduction of such proteins into cells. Furthermore, also protocols for the intracellular expression of proteins are well established.

Proteins have become accessible targets for chemical synthesis. The basic strategy is to use native chemical ligation, Staudinger ligation or related methods (Nilsoon et al., 2005. Chemical synthesis of proteins. Ann. Rev. Biophys. Biomol. Struct. 34, 91-118).

Furthermore, methods for the introduction of peptides and proteins as large as the beta-galactosidase enzyme into cells in culture as well as animal models have been developed (Wadia and Dowdy, 2002. Protein transduction technology. Curr. Opin. Biotechnology 13, 52-56).

Methods for the production of recombinant proteins in prokaryotic and eukaryotic cells are widely established (Chapter 5 Production of Recombinant Proteins in "Current Protocols in Protein Science", Editors: John. E. Coligan, Ben M. Dunn, Hidde L. Ploegh, David W. Speicher, Paul T. Wingfield; Wiley, 1995, ISBN: 0-471-14098-8).

According to the present invention, the term antibody or antibody fragment is also understood as meaning antibodies or antigen-binding parts thereof, which have been prepared recombinantly and, where appropriate, modified, such as chimaeric antibodies, humanized antibodies, multifunctional antibodies, bispecific or oligospecific antibodies, single-stranded antibodies and F(ab) or F(ab)₂ fragments (see, for example, EP-B1-0 368 684, US 4,816,567, US 4,816,397, WO 88/01649, WO 93/06213 or WO 98/24884), preferably produced with the help of a FAB expression library.

As an alternative to the classical antibodies it is also possible, for example, to use protein scaffolds against Pen-2, e.g. anticalins which are based on lipocalin (Beste et al. (1999) Proc. Natl. Acad. Sci. USA, 96, 1898-1903, Skerra, 2000, Biochim. Biophys. Acta, 1482, 337-50, Skerra (2000) J. Mol. Recognit., 13, 167-187).

The procedure for preparing an antibody or antibody fragment is effected in accordance with methods which are well known to the skilled person, e.g. by immunizing a mammal, for example a rabbit, with Pen-2, where appropriate in the presence of, for example, Freund's adjuvant and/or aluminium hydroxide gels (see, for example, Diamond, B.A. et al. (1981) The New England Journal of Medicine: 1344-1349). Monoclonal antibodies can, for example, be prepared in accordance with the known method of Winter & Milstein (Winter, G. & Milstein, C. (1991) Nature, 349, 293-299). General procedures for the production and use of antibodies have been extensively described (Ed Harlow and David Lane: Antibodies. Cold Spring Harbor Laboratory, 1998, ISBN 0-87969-314-2).

Throughout the invention, the term "modulating the activity of gamma secretase" includes that the activity of the enzyme is modulated directly or indirectly. That means that the modulator in binding to Pen-2 may affect gamma-secretase activity either directly, e.g. by triggering a conformational change in Pen-2, or indirectly: The modulator may exert an influence on Pen-2 which in turn, e.g. by protein-protein interactions, allosteric mechanisms and induced conformational changes in general, or by signal transduction or via small metabolites, modulates the activity of gamma secretase.

In the context of the present invention, "modulating the activity of gamma secretase" means that the activity is reduced in that less or no product is formed, most preferably that less or no Abeta-42 is formed, (partial or complete inhibition) or that the respective enzyme produces a different product (e.g. Abeta-38 or other Abeta peptide species of shorter amino acid sequence - instead of Abeta-42) or that the relative quantities of the products are different (e.g. the ratio of Abeta-40 to Abeta-42 is changed, preferably increased).

With respect to the modulator of gamma secretase activity, it is preferred that this modulator inhibits gamma secretase activity. However, it is also preferred that the activity of gamma secretase is shifted in a way that the total amount of Abeta peptide species is unchanged but that more Abeta-38 or other Abeta peptide species of shorter amino acid sequence is produced instead of Abeta-42.

Gamma secretase activity can e.g. measured by determining APP processing, e.g. by determining levels of Abeta peptide species produced, most importantly levels of Abeta-42 (see Example-section, infra).

Preferably, the neurodegenerative disease is Alzheimer's disease.

As explained above, it has been found surprisingly in the context of the present invention that Pen-2 contains a binding site for NSAIDs. Therefore, in a preferred embodiment of the present invention, in step a) a compound is identified which is capable of binding to the Pen-2 binding site characterized by the following amino acid sequence as given in SEQ ID NO:2.

In another preferred embodiment of the method of the invention, in step a) a given test compound is brought into contact with a peptide comprising the Pen-2 binding site as defined in claim 2 or a fragment or homologue thereof.

The method of the invention is preferably performed in the context of a high throughput assay. Such assays are known to the person skilled in the art.

Test or candidate molecules to be screened can be provided as mixtures of a limited number of specified compounds, or as compound libraries, peptide libraries and the like. Agents/molecules to be screened may also include all forms of antisera, antisense nucleic acids, etc., that can modulate complex activity or formation. Exemplary candidate molecules and libraries for screening are set forth below.

Screening the libraries can be accomplished by any of a variety of commonly known methods. See, e.g., the following references, which disclose screening of peptide libraries: Parmley and Smith, 1989, Adv. Exp. Med. Biol. 251:215-218; Scott and Smith, 1990, Science 249:386-390; Fowlkes et al., 1992, BioTechniques 13:422-427; Oldenburg et al., 1992, Proc. Natl. Acad. Sci. USA 89:5393-5397; Yu et al., 1994, Cell 76:933-945; Staudt et al., 1988, Science 241:577-580; Bock et al., 1992, Nature 355:564-566; Tuerk et al., 1992, Proc. Natl. Acad. Sci. USA 89:6988-6992; Ellington et al., 1992, Nature 355:850-852; U.S. Patent No. 5,096,815, U.S. Patent No. 5,223,409, and U.S. Patent No. 5,198,346, all to Ladner et al.; Rebar and Pabo, 1993, Science 263:671-673; and International Patent Publication No. WO 94/18318.

In a specific embodiment, screening can be carried out by contacting the library members with a Pen-2 protein immobilized on a solid phase, and harvesting those library members that bind to the protein (or encoding nucleic acid or derivative). Examples of such screening methods, termed "panning" techniques, are described by way of example in Parmley and Smith, 1988, Gene 73:305-318; Fowlkes et al., 1992, BioTechniques 13:422-427; International Patent Publication No. WO 94/18318; and in references cited hereinabove.

In a specific embodiment, Pen-2-fragments and/or analogs, especially peptidomimetics, are screened for activity as competitive or non-competitive inhibitors of the formation of a complex of PEN-2 with another proteins, e.g. the proteins given in Table 1 (amount of complex or composition of complex) or PEN-2 activity in the cell, which thereby inhibit complex activity or formation in the cell.

In a second common approach to binding assays, one of the binding species is immobilized on a filter, in a microtiter plate well, in a test tube, to a chromatography matrix, etc., either covalently or non-covalently. Proteins can be covalently immobilized using any method well known in the art, for example, but not limited to the method of Kadonaga and Tjian, 1986, Proc. Natl. Acad. Sci. USA 83:5889-5893, i.e., linkage to a cyanogen-bromide derivatized substrate such as CNBr-Sepharose 4B (Pharmacia). Where needed, the use of spacers can reduce steric hindrance by the substrate. Non-covalent attachment of proteins to a substrate include, but are not limited to, attachment of a protein to a charged surface, binding with specific antibodies, binding to a third unrelated interacting protein, etc.

Assays of agents (including cell extracts or a library pool) for competition for binding of one member of a complex (or derivatives thereof) with another member of the complex labeled by any means (e.g., those means described above) are provided to screen for competitors or enhancers of complex formation.

In specific embodiments, blocking agents to inhibit non-specific binding of reagents to other protein components, or absorptive losses of reagents to plastics, immobilization matrices, etc., are included in the assay mixture. Blocking agents include, but are not restricted to bovine serum albumin, casein, nonfat dried milk, Denhardt's reagent, Ficoll, polyvinylpyrolidine, nonionic detergents (NP40, Triton X-100, Tween 20, Tween 80, etc.), ionic detergents (e.g., SDS, LDS, etc.), polyethylene glycol, etc. Appropriate blocking agent concentrations allow complex formation.

After binding is performed, unbound, labeled protein is removed in the supernatant, and the immobilized protein retaining any bound, labeled protein is washed extensively. The amount of bound label is then quantified using standard methods in the art to detect the label as described, supra.

Exemplary assays useful for measuring the production of Abeta-40 and Abeta-42 peptides by ELISA include but are not limited to those described in Vassar R et al., 1999, Science, 286:735-41.

Exemplary assays useful for measuring the production of C-terminal APP fragments in cell lines or transgenic animals by western blot include but are not limited to those described in Yan R et al., 1999, Nature, 402:533-7.

Exemplary assays useful for measuring the proteolytic activity of beta- or gamma secretases towards bacterially expressed APP fragments in vitro (e.g. by modifying the expression of one or several interacting proteins in cells by means of RNAi (siRNA) and/or plasmids encoding the interacting protein(s)) of the BACE1-complex include but are not limited to those described in Tian G et al., 2002, J Biol Chem, 277:31499-505.

Exemplary assays useful for measuring transactivation of a Gal4-driven reporter gene (e.g. by modifying the expression of one or several interacting proteins in cells by means of RNAi (siRNA) and/or plasmids encoding the interacting protein(s)) of the BACE1-complex include but are not limited to those described in Cao X et al., 2001, Science, 293:115-20.

Any molecule known in the art can be tested for its ability to be an interacting molecule or inhibitor according to the present invention. Candidate molecules can be directly provided to a cell expressing the PEN-2-complex machinery, or, in the case of candidate proteins, can be provided by providing their encoding nucleic acids under conditions in which the nucleic acids are recombinantly expressed to produce the candidate protein.

The method of the invention is well suited to screen chemical libraries for molecules which modulate, e.g., inhibit, antagonize, or agonize, the amount of, activity of, or protein component composition of the complex. The chemical libraries can be peptide libraries, peptidomimetic libraries, chemically synthesized libraries, recombinant, e.g., phage display libraries, and in vitro translation-based libraries, other non-peptide synthetic organic libraries, etc.

Exemplary libraries are commercially available from several sources (ArQule, Tripos/PanLabs, ChemDesign, Pharmacopoeia). In some cases, these chemical libraries are generated using combinatorial strategies that encode the identity of each member of the library on a substrate to which the member compound is attached, thus allowing direct and immediate identification of a molecule that is an effective modulator. Thus, in many combinatorial approaches, the position on a plate of a compound specifies that compound's composition. Also, in one example, a single plate position may have from 1-20 chemicals that can be screened by administration to a well containing the interactions of interest. Thus, if modulation is detected, smaller and smaller pools of interacting pairs can be assayed for the modulation activity. By such methods, many candidate molecules can be screened.

Many diversity libraries suitable for use are known in the art and can be used to provide compounds to be tested according to the present invention. Alternatively, libraries can be constructed using standard methods. Chemical (synthetic) libraries, recombinant expression libraries, or polysome-based libraries are exemplary types of libraries that can be used.

The libraries can be constrained or semirigid (having some degree of structural rigidity), or linear or nonconstrained. The library can be a cDNA or genomic expression library, random peptide expression library or a chemically synthesized random peptide library, or non-peptide library. Expression libraries are introduced into the cells in which the assay occurs, where the nucleic acids of the library are expressed to produce their encoded proteins.

In one embodiment, peptide libraries that can be used in the present invention may be libraries that are chemically synthesized in vitro. Examples of such libraries are given in Houghten et al., 1991, Nature 354:84-86, which describes mixtures of free hexapeptides in which the first and second residues in each peptide were individually and specifically defined; Lam et al., 1991, Nature 354:82-84, which describes a "one bead, one peptide" approach in which a solid phase split synthesis scheme produced a library of peptides in which each bead in the collection had immobilized thereon a single, random sequence of amino acid residues; Medynski, 1994, Bio/Technology 12:709-710, which describes split synthesis and T-bag synthesis methods; and Gallop et al., 1994, J. Med. Chem. 37:1233-1251. Simply by way of other examples, a combinatorial library may be prepared for use, according to the methods of Ohlmeyer et al., 1993, Proc. Natl. Acad. Sci. USA 90:10922-10926; Erb et al., 1994, Proc. Natl. Acad. Sci. USA 91:11422-11426; Houghten et al., 1992, Biotechniques 13:412; Jayawickreme et al., 1994, Proc. Natl. Acad. Sci. USA 91:1614-1618; or Salmon et al., 1993, Proc. Natl. Acad. Sci. USA 90:11708-11712. PCT Publication No. WO 93/20242 and Brenner and Lerner, 1992, Proc. Natl. Acad. Sci. USA 89:5381-5383 describe "encoded combinatorial chemical libraries," that contain oligonucleotide identifiers for each chemical polymer library member.

In a preferred embodiment, the library screened is a biological expression library that is a random peptide phage display library, where the random peptides are constrained (e.g., by virtue of having disulfide bonding).

Further, more general, structurally constrained, organic diversity (e.g., nonpeptide) libraries, can also be used. By way of example, a benzodiazepine library (see e.g., Bunin et al., 1994, Proc. Natl. Acad. Sci. USA 91:4708-4712) may be used.

Conformationally constrained libraries that can be used include but are not limited to those containing invariant cysteine residues which, in an oxidizing environment, cross-link by disulfide bonds to form cystines, modified peptides (e.g., incorporating fluorine, metals, isotopic labels, are phosphorylated, etc.), peptides containing one or more non-naturally occurring amino acids, non-peptide structures, and peptides containing a significant fraction of -carboxyglutamic acid.

Libraries of non-peptides, e.g., peptide derivatives (for example, that contain one or more non-naturally occurring amino acids) can also be used. One example of these are peptoid libraries (Simon et al., 1992, Proc. Natl. Acad. Sci. USA 89:9367-9371). Peptoids are polymers of non-natural amino acids that have naturally occurring side chains attached not to the alpha-carbon but to the backbone amino nitrogen. Since peptoids are not easily degraded by human digestive enzymes, they are advantageously more easily adaptable to drug use. Another example of a library that can be used, in which the amide functionalities in peptides have been permethylated to generate a chemically transformed combinatorial library, is described by Ostresh et al., 1994, Proc. Natl. Acad. Sci. USA 91:11138-11142).

The members of the peptide libraries that can be screened according to the invention are not limited to containing the 20 naturally occurring amino acids. In particular, chemically synthesized libraries and polysome based libraries allow the use of amino acids in addition to the 20 naturally occurring amino acids (by their inclusion in the precursor pool of amino acids used in library production). In specific embodiments, the library members contain one or more non-natural or non-classical amino acids or cyclic peptides. Non-classical amino acids include but are not limited to the D-isomers of the common amino acids, -amino isobutyric acid, 4-aminobutyric acid, Abu, 2-amino butyric acid; .-Abu,.-Ahx, 6-amino hexanoic acid; Aib, 2-amino isobutyric acid; 3-amino propionic acid; ornithine; norleucine; norvaline, hydroxyproline, sarcosine, citrulline, cysteic acid, t-butylglycine, t-butylalanine, phenylglycine, cyclohexylalanine, β-alanine, designer amino acids such as β-methyl amino acids, C-methyl amino acids, N-methyl amino acids, fluoro-amino acids and amino acid analogs in general. Furthermore, the amino acid can be D (dextrorotary) or L (levorotary).

In another embodiment of the present invention, combinatorial chemistry can be used to identify modulators of a the complexes. Combinatorial chemistry is capable of creating libraries containing hundreds of thousands of compounds, many of which may be structurally similar. While high throughput screening programs are capable of screening these vast libraries for affinity for known targets, new approaches have been developed that achieve libraries of smaller dimension but which provide maximum chemical diversity. (See e.g., Matter, 1997, J. Med. Chem. 40:1219-1229).

One method of combinatorial chemistry, affinity fingerprinting, has previously been used to test a discrete library of small molecules for binding affinities for a defined panel of proteins. The fingerprints obtained by the screen are used to predict the affinity of the individual library members for other proteins or receptors of interest (in the instant invention, the protein complexes of the present invention and protein components thereof.) The fingerprints are compared with fingerprints obtained from other compounds known to react with the protein of interest to predict whether the library compound might similarly react. For example, rather than testing every ligand in a large library for interaction with a complex or protein component, only those ligands having a fingerprint similar to other compounds known to have that activity could be tested. (See, e.g., Kauvar et al., 1995, Chem. Biol. 2:107-118; Kauvar, 1995, Affinity fingerprinting, Pharmaceutical Manufacturing International. 8:25-28; and Kauvar, Toxic-Chemical Detection by Pattern Recognition in New Frontiers in Agrochemical Immunoassay, Kurtz, Stanker and Skerritt (eds), 1995, AOAC: Washington, D.C., 305-312).

Kay et al. (1993, Gene 128:59-65) disclosed a method of constructing peptide libraries that encode peptides of totally random sequence that are longer than those of any prior conventional libraries. The libraries disclosed in Kay et al. encode totally synthetic random peptides of greater than about 20 amino acids in length. Such libraries can be advantageously screened to identify complex modulators. (See also U.S. Patent No. 5,498,538 dated March 12, 1996; and PCT Publication No. WO 94/18318 dated August 18, 1994).

A comprehensive review of various types of peptide libraries can be found in Gallop et al., 1994, J. Med. Chem. 37:1233-1251.

Preferably, it is determined in the context of the present invention whether the test compound is able to bind to the peptide comprising the Pen-2 binding site as defined above or a fragment or homologue thereof.

The peptide comprising the Pen-2 binding site or the fragment or homologue thereof may be a purified protein, part of a membrane preparation or expressed in a cell (on the surface or in a cell compartment).

Therefore, the test compound may be brought into contact with purified PEN-2 protein. Methods for the purification of proteins, including membrane proteins, belong to the standard repertoire of biochemistry laboratories.

Furthermore, the method of the present invention may be performed by bringing the test compound is brought into contact with isolated late endosomes/lysosomes containing PEN-2, and the interaction of PEN-2 with the test compound is determined. General methods how to isolate cell organelles are widely known in the art (Chapter 4.2 Purification of Organelles from Mammalian Cells in ""Current Protocols in Protein Science", Editors: John.E. Coligan, Ben M. Dunn, Hidde L. Ploegh, David W. Speicher, Paul T. Wingfield; Wiley, ISBN: 0-471-14098-8). In addition, specific methods for the purification of late endosomes/ lysosomes on density gradients by ultracentrifugation have been described (Wunderlich et al., 2001, The Journal of Cell Biology 152, 765-776). The source of endodomes/lysosomes can either be cells that express endogenous PEN-2 protein or cells that are transfected with PEN-2 expression vectors and produce recombinant PEN-2 protein or suitable fragments thereof.

Additionally, the test compound is brought into contact with isolated membrane fractions containing PEN-2, and the interaction of PEN-2 with the test compound is determined. Membrane fractions can be prepared by fractionation of cell extracts thereby enriching specific types of proteins such as membrane proteins (Chapter 4.3 Subcellular Fractionation of Tissue Culture Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

Furthermore, the test compound may be brought into contact with whole cells expressing the peptide.

In general, methods for the expression of proteins in cells are known in the art.

Preferably, the method of the invention is performed in the absence of other proteins of the the gamma secretase complex, more preferably in the absence of Aph 1, Aph 2 and/or Pen-1, more preferably in the absence of these three proteins. This may be e.g. achieved by using a cell line for the expression pf Pen-2 which is incapable of expressing Aph 1, Aph 2 and/or Pen-1.

In a preferred embodiment of the method of the invention, the peptide comprising the Pen-2 binding site is Pen-2.

Preferably, the ability to bind is tested by using a competitive binding assay, preferably by using a ligand, more preferably a gamma-secretase modulating compound, even more preferred a gamma-secretase modulating compound with a binding constant of less than 10 µM, even more preferred less than 1 µM, most preferred less than 100 nM.

The ligand can be radioactively labeled or labeled with a fluorescent dye or with biotin or another moiety enabling detection means utilizing principles of biomolecular recognition. Methods for labeling ligands with fluorescent dyes or with biotin are known in the art. For example, commercially-available radioactively labeled Flurbiprofen can be used.

According to the invention, the PEN-2 interacting compound identified in step a) is then subjected to process step b). This step includes the determination whether the Pen-2 interacting compound is able to modulate gamma-secretase activity.

According to a preferred embodiment, in step b) the ability of the gamma-secretase to cleave APP is measured. This can be measured as indicated above.

More preferably, the ability of gamma secretase to produce Abeta 42 from APP is measured.

As explained above, the gamma-secretase complex comprises different proteins. It has been found in the context of the present invention that Pen-2 contains a binding site for NSAIDs, which modulate the activity of the gamma-secretase complex. However, the catalytic site of the complex is located elsewhere, namly on PS.

Therefore, the identification of the NSAID binding site on Pen-2 enables the identification of molecules which bind to the catalytic site and have therefore inhibitory properties, but which do not bind to Pen-2 binding site.

Consequently, in a further aspect, the invention relates to a method for the identification of a gamma secretase inhibitor, comprising the steps of
a) providing a gamma-secretase protein complex comprising a mutated Pen-2 protein with a non functional Pen-2 binding site,
b) contacting the gamma-secretase protein complex as defined in step a) with a test compound, and
c) determining whether the test compound inhibits gamma-secretase activity.

All embodiments described above which are applicable for this method of the invention also apply here.

A "non functional Pen-2 binding site" is a binding site unable to bind a Pen-2 interacting compound.

Preferably, the proteins of the gamma-secretase protein complex defined in step a) have been expressed in a cell, preferably the same cell.

More preferably, the cell is unable to express functional endogenous Pen-2. The production of such cells is known in the art.

A suitable stable cell line in which the endogenous Pen-2 protein level is downregulated via RNA interference-mediated Pen-2 knockdown was described (Prokop et al., 2004. J. Biol. Chem. 279, 23255- 23261). This cell line can be transfected with cDNA constructs encoding RNAi-rsistant wildtype or mutant Pen-2 proteins to test their functional activity.

In a preferred embodiment, in step c) the ability of a test compound to inhibit APP cleavage is determined as explained above.

The invention also encompasses a method wherein in addition to the method of the invention above using a protein complex with a mutated Pen-2 gene also a protein complex is used which has a Pen-2 with the Pen-2 binding site. Both complexes are then contacted with the test compound, and an inhibitor is identified by lowering Abeta42 in cells expressing wt Pen-2 but not in cells expressing Pen-2 with a non functional Pen-2 binding site.

The invention further relates to a method for preparing a pharmaceutical composition for the treatment of neurodegenerative diseases, comprising the following steps:
a) identifying a gamma-secretase modulator according to any of claims 1 to 11 or a gamma-secretase inhibitor according to any of claims 12 to 14 , and
b) formulating the gamma-secretase modulator or inhibitor to a pharmaceutical composition.

According to the invention, the Pen-2 interacting compound which is a gamma-secretase modulator or inhibitor may be used to prepare a pharmaceutical composition.

Therefore, the invention provides pharmaceutical compositions, which may be administered to a subject in an effective amount. In a preferred aspect, the therapeutic is substantially purified. The subject is preferably an animal including, but not limited to animals such as cows, pigs, horses, chickens, cats, dogs, etc., and is preferably a mammal, and most preferably human. In a specific embodiment, a non-human mammal is the subject.

Various delivery systems are known and can be used to administer a therapeutic of the invention, e.g., encapsulation in liposomes, microparticles, and microcapsules: use of recombinant cells capable of expressing the therapeutic, use of receptor-mediated endocytosis (e.g., Wu and Wu, 1987, J. Biol. Chem. 262:4429-4432); construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions of the invention locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the therapeutic can be delivered in a vesicle, in particular a liposome (Langer, 1990, Science 249:1527-1533; Treat et al., 1989, In: Liposomes in the Therapy of Infectious Disease and Cancer, Lopez-Berestein and Fidler, eds., Liss, New York, pp. 353-365; Lopez-Berestein, ibid., pp. 317-327; see generally ibid.)

In yet another embodiment, the therapeutic can be delivered via a controlled release system. In one embodiment, a pump may be used (Langer, supra; Sefton, 1987, CRC Crit. Ref. Biomed. Eng. 14:201-240; Buchwald et al., 1980, Surgery 88:507-516; Saudek et al., 1989, N. Engl. J. Med. 321:574-579). In another embodiment, polymeric materials can be used (Medical Applications of Controlled Release, Langer and Wise, eds., CRC Press, Boca Raton, Florida, 1974; Controlled Drug Bioavailability, Drug Product Design and Performance, Smolen and Ball, eds., Wiley, New York, 1984; Ranger and Peppas, 1983, Macromol. Sci. Rev. Macromol. Chem. 23:61; Levy et al., 1985, Science 228:190-192; During et al., 1989, Ann. Neurol. 25:351-356; Howard et al., 1989, J. Neurosurg. 71:858-863). In yet another embodiment, a controlled release system can be placed in proximity of the therapeutic target, i.e., the brain, thus requiring only a fraction of the systemic dose (e.g., Goodson, 1984, In: Medical Applications of Controlled Release, supra, Vol. 2, pp. 115-138). Other controlled release systems are discussed in the review by Langer (1990, Science 249:1527-1533).

In a specific embodiment where the therapeutic is a nucleic acid, preferably encoding a protein therapeutic, the nucleic acid can be administered in vivo to promote expression of its encoded protein, by constructing it as part of an appropriate nucleic acid expression vector and administering it so that it becomes intracellular, e.g., by use of a retroviral vector (U.S. Patent No. 4,980,286), or by direct injection, or by use of microparticle bombardment (e.g., a gene gun; Biolistic, Dupont), or by coating it with lipids, cell-surface receptors or transfecting agents, or by administering it in linkage to a homeobox-like peptide which is known to enter the nucleus (e.g., Joliot et al., 1991, Proc. Natl. Acad. Sci. USA 88:1864-1868), etc. Alternatively, a nucleic acid therapeutic can be introduced intracellularly and incorporated by homologous recombination within host cell DNA for expression.

As mentioned above, also the binding proteins can be expressed intracellularly. This implies that at least a nucleic acid encoding the binding protein is formulated in a suitable way (as indicated above) and administered to the patient/subject in a way (also see above) which enables the intracellular expression of the binding protein.

In general, the pharmaceutical compositions of the present invention comprise a therapeutically effective amount of a therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc.. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc.

The amount of the therapeutic of the invention which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems.

Suppositories generally contain active ingredient in the range of 0.5% to 10% by weight; oral formulations preferably contain 10% to 95% active ingredient.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The kits of the present invention can also contain expression vectors encoding the essential components of the complex machinery, which components after being expressed can be reconstituted in order to form a biologically active complex. Such a kit preferably also contains the required buffers and reagents. Optionally associated with such container(s) can be instructions for use of the kit and/or a notice in the form prescribed by a governmental agency regulating the manufacture, use or sale of pharmaceuticals or biological products, which notice reflects approval by the agency of manufacture, use or sale for human administration.

As explained above, the method of the invention for the preparation of a pharmaceutical composition may further comprise the step of mixing the identified compound with a pharmaceutically acceptable carrier.

The invention further relates to a pharmaceutical composition comprising the gamma secretase modulator or inhibitor identified according to the methods of the invention.

Furthermore, the invention relates to a pharmaceutical composition obtainable by the method of the invention for the preparation of a pharmaceutical composition.

Preferably, the pharmaceutical compositions of the invention are for the treatment of neurodegenerative disease such as Alzheimer's disease and related neurodegenerative disorders, preferably Alzheimer's disease.

Furthermore, the invention relates to the use of the above pharmaceutical composition for the treatment of neurodegenerative disease such as Alzheimer's disease and related neurodegenerative disorders. With respect to this pharamceutical composition and its administration, the embodiments as described above also apply.

Furthermore, the invention also refers to a method for treating or preventing a neurodegenerative disease, preferably Alzheimer's disease administering to a subject in need of such treatment or prevention a therapeutically effective amount of the above pharmaceutical composition. Also with respect to this method of the invention, the embodiments described above with respect to the pharmaceutical composition of the invention also apply.

Recent publications suggested that gamma-secretase modulators developed for Alzheimer's disease, might be of therapeutic benefit in colorectal neoplastic diseases (van Es et al., 2005, Notch/gamma-secreatse inhibition turns proliferative cells in the intestinal crypts and adenomas into goblet cells. Nature 435, 959-963). In addition to the APP protein a number of other membrane proteins serve as substrate for gamma-secretase cleavage. One class of substrates are the Notch proteins which regulate cell-fate decisions. Notch signaling is involved in controlling the fate of intestinal epithelial tissue. Interestingly, blockade of the Notch pathway by the gamma-secretase inhibitor DBZ stopped growth of adenomas (polyps) in the the small intestine and colon (van Es and Clevers, 2005. Notch and Wnt inhibitors as potential new drugs for intestinal neoplastic disease. Trends in Molecular Medicine 11, 496-502). Independenly, it was reported that gamma-secretase inhibitors may be of therapeutic use in Kaposi's sarcoma patients (Curry et al., 2005. Gamma secretase inhibitor blocks Notch activation and induces apoptosis in Kaposi's sarcoma tumor. Oncogne 24, 6333-6344).

Consequently, the invention also relates to the pharmaceutical composition as described above for the treatment of cancer, preferably colon cancer.

Furthermore, the invention relates to the use of the above pharmaceutical composition for the treatment of cancer, preferably colon cancer.

The invention also refers to a method for treating or preventing cancer, preferably colon cancer administering to a subject in need of such treatment or prevention a therapeutically effective amount of the above pharmaceutical composition.

The embodiments as described above for the other methods and uses of the invention also apply to this method and use in the context of tumor therapy. In the case of cancer therapy, oral administration is preferred.

The following examples and figures will describe the subject-matter of the invention in more detail.

### Short description of the figures

### Figure 1: Sequence alignment of Cox enzymes and human Pen-2

The sequences of the carboxy-terminal parts of human cyclooxygenase-2 (COX-2_Hum_C-term), sheep cyclooxygenase-2 (1CX-2_Sheep_C-term) and human cyclooxygenase-1 (COX-1_Hum_C-term) were aligned with the full-length human Pen-2 protein sequence using aligment programs and further adjusted manually. Amino acids that are identical in all four sequences are highlighted in bold and indicated by the # symbol above the alignment.
The amino acid residues in Cox-1 implicated by X-ray crystallography in interaction with S-Flurbiprofen are indicated by an asterisk (Picot D, Loll PJ, Garavito RM, 1994. The X-ray crystal structure of the membrane protein prostaglandin H2 synthase-1. Nature 367, 243-249).
The Pen-2 sequence comprising the predicted "Pen-2 binding motif" for NSAIDs is underlined (amino acid residues 1 to 59 of human Pen-2).
Within this sequence are two short conserved sequence elements that are predicted to be important for NSAID binding (Box 1: Y19 to L26 in PEN-2, YLGGFAFL; Box 2: Q52 to R59, QIKGYVWR in Pen-2).

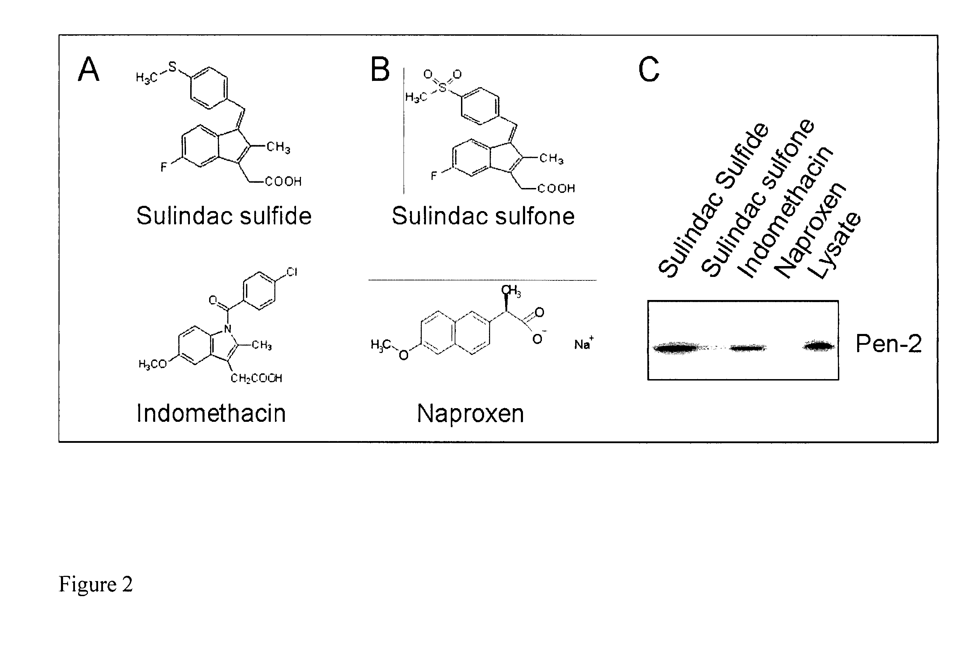

### Figure 2: Pen-2 binds to immobilized GSMs but not to non-GSMs

Non-steroidal anti-inflammatory carboxylic acids with known gamma-secretase modulatory activity (GSM, Sulindac sulfide and Indomethacin, Figure 2 A) or confirmed lack thereof (non-GSM, Sulindac sulfone and Naproxen, Figure 2 B) were tested in compound pull-down experiments (Figure 2 C).
Compounds were covalently linked to sepharose beads through their carboxylic acid groups and remaining reactive sites on sepharose were blocked. A cell lysate was prepared from a SK-N-BE2 neuroblastoma cell line, expressing TAP-tagged presenilin1 and HA-tagged gamma -secretase subunits nicastrin, Aph1a and Pen-2, by lysis in a buffer containing 1% (w/v) CHAPSO. The lysate was incubated with Sepharose-bound compounds. After extensive washing, bound proteins were eluted in SDS-PAGE sample buffer and analyzed by gel electrophoresis and Western blotting with a HA-tag specific antibody.

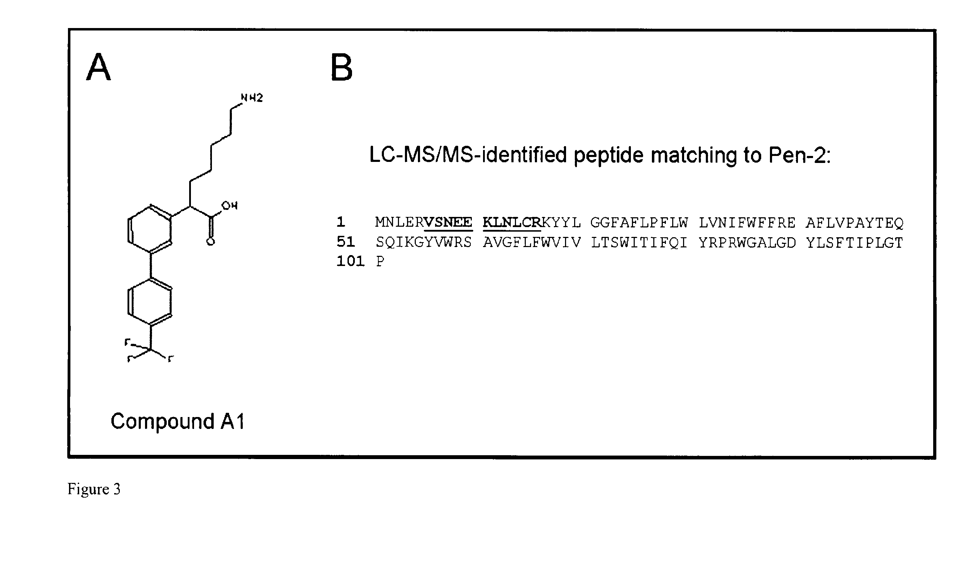

### Figure 3: An immobilized amine-containing analog of a gamma-secretase modulator identifies Pen-2 in drug pulldown experiments

The amine-containing analog of a gamma-secretase modulator, Compound A1, (A), was immobilized on NHS-Sepharose. Lysate of a gamma-secretase-expressing SK-N-BE2 neuroblastoma cell line was incubated with the beads, which were then washed extensively and finally eluted with SDS-PAGE sample buffer. Samples were alkylated with iodoacetamide and separated on a 4-12% Bis-Tris gel. The entire gel lane was cut into pieces, all of which were treated with trypsin, desalted and further processed for LC-MS/MS analysis and MASCOT database search. A peptide matching to Pen-2 (sequence underlined in B) was identified.

### Figure 4

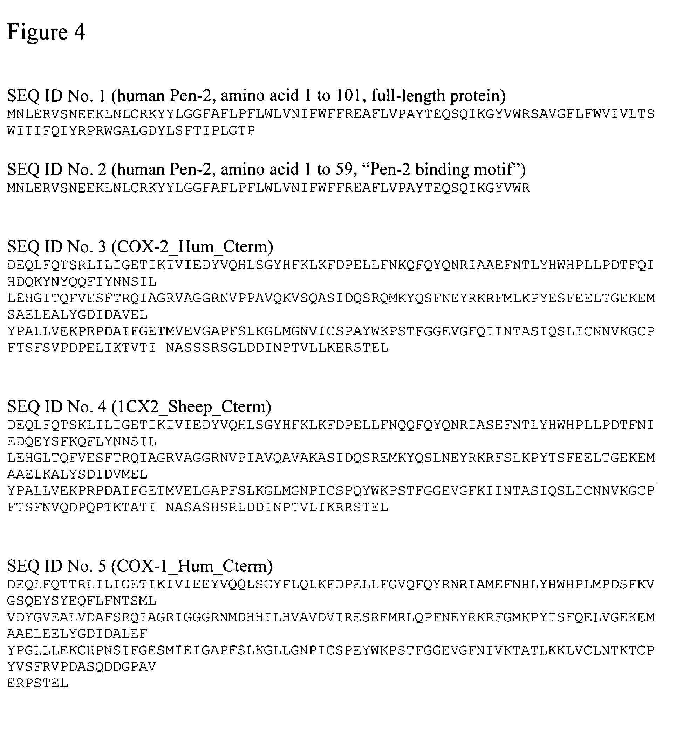

Figure 4 shows different individual sequences important in the contxt of the present invention.

### Examples

### Example 1: Sequence and Structure Analysis of human Pen-2

The sequences of the carboxy-terminal parts of human cyclooxygenase-2, sheep cyclooxygenase-2 and human cyclooxygenase-1 were aligned with the full-length human Pen-2 protein sequence using publicly accessible alignment programs and further adjusted manually (Figure 1). Furthermore, the amino acid residues in Cox-1 implicated by X-ray crystallography in interaction with the NSAID S-Flurbiprofen were taken into account (Picot D, Loll PJ, Garavito RM, 1994. The X-ray crystal structure of the membrane protein prostaglandin H2 synthase-1. Nature 367, 243-249).
This analysis identifies an NSAID binding motif within the human Pen-2 protein - comprising amino acid residues 1 to 59 ("Pen-2 binding motif', underligned in Figure 1). Furthermore, within this motif the analysis predicts two short conserved sequence elements (Box 1: Y19 to L26 in PEN-2, YLGGFAFL; Box 2: Q52 to R59, QIKGYVWR in Pen-2) as well as specific amino acids (indicated by asterisks in Figure 1) that are responsible for directly contacting the NSAID. Mutation of the amino acids predicted to directly contact an NSAID (see Example 4) can therefore be used to generate Pen-2 variants devoid of GSM-binding activity as well as to differentiate a γ-secretase modulator from a γ-secretase inhibitor (see Example 5).
In contrast, the analysis did not reveal any sequence conservation between Cox-1 and Pen-2 in stretches of the sequence which correspond to the peroxidase active site of Cox-1. Likewise, no sequence conservation was detected between Cox-1 and Pen-2 in that part of the Cox-1 sequence which corresponds to the EGF-like motif.
The amino acids of Cox-1 found within box 1 form one side of the arachidonic acid / NSAID binding channel leading to the cyclooxygenase active site; the amino acids of Cox-1 within box 2 form the apex of the arachidonic acid / NSAID binding channel adjacent to the cyclooxygenase active site.
The amino acids of Pen-2 within boxes I and 2 are predicted to not only form part of the NSAID binding pocket, but would also be predicted to provide direct contacts to the NSAID itself.

### Example 2: Selectivity of binding of NSAIDS to Pen-2

A number of NSAIDs, all of them carboxylic acids whose gamma-secretase modulatory activity or lack thereof had been reported previously, were tested for their ability to bind Pen-2. (Weggen et al., 2001. A subset of NSAIDs lower amyloidogenic Abeta42 independently of cyclooxygenase activity. Nature 414, 212-216; Eriksen et al., 2003. NSAIDs and enantiomers of flurbiprofen target gamma-secretase and lower Abeta 42 in vivo. J. Clin. Invest. 112, 440-449).

To this end, the compounds were covalently attached to sepharose beads via their carboxylic acid groups. Coupling efficiency was confirmed to be greater than 90 % by HPLC analysis and potential reactive sites remaining on beads were blocked by incubation with N-hydroxysuccinimide acetate. Compound-coupled beads were used for drug pulldown experiments. Pools of SK-N-BE2 neuroblastoma cells, expressing tandem affinity purification (TAP-) tagged presenilin I as well as HA-tagged gamma-secretase subunits nicastrin, Aph1a and Pen-2, were generated by retroviral transduction and propagated in appropriate cell culture medium. Cells were then lysed in a buffer containing 1% (w/v) CHAPSO detergent and 0.5 mg lysate per pull-down experiment was incubated with Sepharose-bound compounds. After extensive washing, bound proteins were eluted in SDS-PAGE sample buffer and analyzed by gel electrophoresis and standard Western blotting with an HA-tag specific antibody.

The result of this experiment shows that NSAIDs with known Abeta42-lowering activity (Sulindac sulfide and Indomethacin) were able to pull down Pen-2 via direct or indirect interaction, whereas NSAIDs lacking Abeta42 lowering acivity (Sulindac sulfone and Naproxen) did not identify Pen-2 (Figure 2).

### Experimental procedures

### Part 1: Immobilization of compounds and drug pull-down experiments

### 1.1. Immobilization of carboxylic acids (N,N'-dicyclohexylcarbodiimide procedure)

The compounds were coupled to reversed NHS-Sepharose beads (DCCD chemistry) as outlined below.

Reversal of beads. NHS-activated Sepharose 4 Fast Flow (Amersham Biosciences, 17-0906-01) was washed 3 times with 10 mL DMSO (Dimethylsulfoxide, Fluka, 41648, H20 <= 0.005%). A mixture containing aminoethanol (2-Aminoethanol, Aldrich, 11.016-7) (160 µmol total), ethylenediamine (Fluka, 03350) (40 µmol total) and triethylamine (TEA) (SIGMA, T-0886, 99% pure) (108 µmol total) was then added to 1 mL settled, washed beads and incubated overnight at room temperature on an end-over-end shaker.

Activation of the COOH group for coupling. After separately dissolving both NHS (N-hydroxy-succinimide, Aldrich, 13,067-2) and DCCD (N,N'-dicyclohexylcarbodiimide, Aldrich, D8,000-2) to 100mM in acetonitrile (Merck, 1.00030.2500), 50 µL of each solution was added to 350 µL acetonitrile. To this mixture, 50 µL 100mM of the COOH containing compound to be coupled was added. Components were gently mixed and stored away from light at room temperature overnight.

Coupling of the activated compound to the reversed NHS beads. After extensive washing of the reversed beads, to remove all excess aminoethanol and ethylene diamine, beads were resuspended in 1 mL DMSO (50% slurry) in a 15mL Falcon tube. 100 µL of the activated compound (above) was added to the beads as well as 15 µL 7.2 M TEA. The coupling reaction was incubated overnight at room temperature on an end-over-end shaker. The coupling efficiency was determined by HPLC. Non-reacted amine-groups were blocked by incubation with 100 µL 100 mM NHS-activated acetic acid at room temperature on the end-over-end shaker over night. Washed beads were stored in isopropanol or immediately used for binding experiments.

**Table 1: Abbreviations used in coupling protocols**

| | |
|---|---|
| DCCD | Dicyclohexylcarbodiimide |
| DMSO | Dimethyl sulfoxide |
| NHS | N-hydroxy-succinimide |
| TEA | Triethylamine |

### 1.2. Preparation of cell lysates

SKNBE2 neuroblastoma cells that express tandem affinity purification (TAP-) tagged presenilin I and HA-tagged gamma-secretase subunits nicastrin, Aph 1a and Pen-2 were homogenized in a Potter S homogenizer (5 ml per 10^8 cells): 50 mM Tris-HCl, 1% CHAPSO, 5% glycerol, 150 mM NaCl, 1.5 mM MgCl2, 25 mM NaF, 1 mM sodium vanadate, 1 mM DTT, pH 7.5 + 1 complete EDTA-free tablet (protease inhibitor cocktail, Roche Diagnostics, 1 873 580) per 25 ml buffer. Material was dounced 10x using a mechanized POTTER S, transferred to 50 ml falcon tubes, incubated for 30 minutes on ice and spun down for 10 min at 20,000 g at 4°C (10,000 rpm in Sorvall SLA600, precooled). The supernatant was transfered to an UZ-polycarbonate tube (Beckmann, 355654) and spun for 1 hour at 100.000 g at 4°C (33.500 rpm in Ti50.2, precooled). The supernatant was transferred again to a fresh 50 ml falcon tube, the protein concentration was determined by a Bradford assay (BioRad) and samples containing 50 mg of protein per aliquot were prepared and immediately used for experiments (see section 1.3) or stored frozen.

### 1.3 Compound pull-down experiment

Sepharose-beads with immobilized compound were equilibrated in lysis buffer and incubated with a cell lysate sample containing 50 mg of protein on an end-over-end shaker (Roto Shake Genie, Scientific Industries Inc.) for 2 hours at 4°C. Beads were collected, transfered to Mobicol-columns (MoBiTech 10055) and washed with 10 ml lysis buffer containing 0.5% detergent, followed by 5 ml lysis buffer with 0.25 % detergent. To elute the bound protein, 60 µl 2 x sample buffer was added, the column was heated for 30 minutes at 50°C and the eluate was transferred to a microfuge tube by centrifugation.

### Part 2: Generation of a cell line expressing HA-tagged gamma-secretase subunits

As a vector, a MoMLV-based recombinant virus was used. The preparation has been carried out as follows:

### 2.1. Preparation of Virus

293 gp cells were grown to 100% confluency. They were split 1:5 on poly-L-Lysine plates (1:5 diluted poly-L-Lysine [0.01% stock solution, Sigma P-4832] in PBS, left on plates for at least 10 min.). On Day 2, 63 microgram of retroviral Vector DNA together with 13 microgram of DNA of plasmid encoding an appropriate envelope protein were transfected into 293 gp cells (Somia, et al., 1999, Proc. Natl. Acad. Sci. USA 96:12667-12672; Somia, et al. 2000, J. Virol. 74:4420-4424). On Day 3, the medium was replaced with 15 ml DMEM + 10% FBS per 15-cm dish. On Day 4, the medium containing viruses (supernatant) was harvested (at 24 h following medium change after transfection). When a second collection was planned, DMEM 10 % FBS was added to the plates and the plates were incubated for another 24 h. All collections were done as follows: The supernatant was filtered through 0.45 micrometer filter (Corning GmbH, cellulose acetate, 431155). The filter was placed into konical polyallomer centrifuge tubes (Beckman, 358126) that are placed in buckets of a SW 28 rotor (Beckman). The filtered supernatant was ultracentrifuged at 19400 rpm in the SW 28 rotor, for 2 hours at 21 degree Celsius. The supernatant was discarded. The pellet containing viruses was resuspended in a small volume (for example 300 microliter) of Hank's Balanced Salt Solution [Gibco BRL, 14025-092], by pipetting up and down 100-times, using an aerosol-safe tip. The viruses were used for transfection as described below.

### 2.2. Infection

Cells that were infected were plated one day before into one well of a 6-well plate. 4 hours before infection, the old medium on the cells was replaced with fresh medium. Only a minimal volume was added, so that the cells are completely covered (e.g. 700 microliter). During infection, the cells were actively dividing.
A description of the cells and their growth conditions is given further below ("2.3. Cell lines"). To the concentrated virus, polybrene (Hexadimethrine Bromide; Sigma, H 9268) was added to achieve a final concentration of 8 microgram/ml (this is equivalent to 2.4 microliter of the I milligram/ml polybrene stock per 300 microliter of concentrated retrovirus). The virus was incubated in polybrene at room temperature for I hour. For infection, the virus/polybrene mixture was added to the cells and incubated at 37 degree Celsius at the appropriate CO2 concentration for several hours (e.g. over-day or over-night). Following infection, the medium on the infected cells was replaced with fresh medium. The cells were passaged as usual after they became confluent. The cells contain the retrovirus integrated into their chromosomes and stably express the gene of interest.

### 2.3. Cell lines

For expression, SKN-BE2 cells were used. SKN-BE2 cells (American Type Culture Collection-No. CRL-2271) were grown in 95% OptiMEM + 5% iron-supplemented calf serum.

### Example 3: Drug pulldown experiments with an amine-containing gamma-secretase modulator (GSM)

Proteins binding to immobilized gamma-secretase modulator compounds were identified using a chemical proteomics procedure (WO04/099774). Binding proteins were purified on resins to which amine compounds, such as compound Al, were covalently attached. Bound proteins were eluted and subsequently separated by SDS-Polyacrylamide gel elecrophoresis, suitable gel bands were cut out and analyzed by LC-MS/MS mass spectrometry.

The results show that the Pen-2 protein can be identified using the immobilized gamma-secretase modulator compounds sulindac sulfide and compound A1.

### 1. Immobilization of small molecule amine compounds

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads were placed in a 15 ml Falcon tube, compound stock solution (usually 100 mM in DMF or DMSO) was added (final conc. 0.2-2 µmol/ml beads) as well as 15 µl of TEA. Beads were incubated at RT in darkness on an end-over-end shaker (Roto Shake Genie, Scientific Industries Inc.) for 16 - 20 h. Coupling efficiency was determined by HPLC. Non-reacted NHS-groups were blocked by incubation with aminoethanol at RT on the end-over-end shaker over night. Washed beads were stored in isopropanol.

### 2. Preparation of cell lysates

Cell lysates were prepared as described in Example 2.

### 3. Protein Identification by Mass Spectrometry

### 3.1 Protein digestion prior to mass spectrometric analysis

Gel-separated proteins were reduced, alkylated and digested in gel essentially following the procedure described by Shevchenko et al., 1996, Anal. Chem. 68:850-858. Briefly, gel-separated proteins were excised from the gel using a clean scalpel, reduced using 10 mM DTT (in 5mM ammonium bicarbonate, 54°C, 45 min) and subsequently alkylated with 55 mM iodoacetamid (in 5 mM ammonium bicarbonate) at room temperature in the dark (30 min). Reduced and alkylated proteins were digested in gel with porcine trypsin (Promega) at a protease concentration of 12.5 ng/µl in 5mM ammonium bicarbonate. Digestion was allowed to proceed for 4 hours at 37°C and the reaction was subsequently stopped using 5 µl 5% formic acid.

### 3.2 Sample preparation prior to analysis by mass spectrometry

Gel plugs were extracted twice with 20 µl 1% TFA and pooled with acidified digest supernatants. Samples were dried in a a vaccuum centrifuge and resuspended in 13 µl 1% TFA.

### 3.3. Mass spectrometric data acquisition

Peptide samples were injected into a nano LC system (CapLC, Waters or Ultimate, Dionex) which was directly coupled either to a quadrupole TOF (QTOF2, QTOF Ultima, QTOF Micro, Micromass) or ion trap (LCQ Deca XP) mass spectrometer. Peptides were separated on the LC system using a gradient of aqueous and organic solvents (see below). Solvent A was 5% acetonitrile in 0.5% formic acid and solvent B was 70% acetonitrile in 0.5% formic acid.

**Table 2: Peptides eluting off the LC system were partially sequenced within the mass spectrometer.**

| Time (min) | % solvent A | % solvent B |
|---|---|---|
| 0 | 95 | 5 |
| 5.33 | 92 | 8 |
| 35 | 50 | 50 |
| 36 | 20 | 80 |
| 40 | 20 | 80 |
| 41 | 95 | 5 |
| 50 | 95 | 5 |

### 3.4. Protein identification

The peptide mass and fragmentation data generated in the LC-MS/MS experiments were used to query fasta formatted protein and nucleotide sequence databases maintained and updated regularly at the NCBI (for the NCBInr, dbEST and the human and mouse genomes) and European Bioinformatics Institute (EBI, for the human, mouse, D. melanogaster and C. elegans proteome databases). Proteins were identified by correlating the measured peptide mass and fragmentation data with the same data computed from the entries in the database using the software tool Mascot (Matrix Science; Perkins et al., 1999. Probability-based protein identification by searching sequence databases using mass spectrometry data. Electrophoresis 20, 3551-3567). Search criteria varied depending on which mass spectrometer was used for the analysis.

### Example 4: Construction of point mutations in the Pen-2 binding motif

In vitro mutagenesis was performed using a commercially available kit and following the instructions of the manufacturer (QuikChange Site-Directed Mutagenesis Kit, catalogue number 200518, Stratagene, San Diego).

The following four Pen-2 mutants were constructed in the context of full-length human Pen-2 (underlined nucleotides indicate position of the mutation):
- Phenylalanine to alanine at position 23 (F23A).
- Tyrosine to alanine at position 56 (Y56A).
- Tryptophan to alanine at position 58 (W58A).
- Arginine to alanine at position 59 (R59A)

Oligonucleotide sequences used for in vitro mutagenesis:

| | | |
|---|---|---|
| F23A: | top oligo | 5' CTGGGGGGGGCTGCTTTCCTG 3' |
| | bottom oligo | 5' CAGGAAAGCAGCCCCCCCCAG 3' |
| | | |
| Y56A: | top oligo | 5' CAAATCAAAGGCGCTGTCTGGCGC 3' |
| | bottom oligo | 5' GCGCCAGACAGCGCCTTTGATTTG 3' |
| | | |
| W58A: | top oligo | 5' GGCTATGTCGCGCGCTCAGCTG 3' |
| | bottom oligo | 5' CAGCTGAGCGCGCGACATAGCC 3' |
| | | |
| R59A: | top oligo | 5'- CTATGTCTGGGCCTCAGCTGTG 3' |
| | botton oligo | 5' CACAGCTGAGGCCCAGACATAG 3' |

Using the described DNA oligonucleotides and pDONR201 vector (Invitrogen, 11798-014) containing wild-type human Pen-2 as a template, a PCR reaction was performed to incorporate the desired DNA mutations into the human Pen-2 gene. After the PCR reaction, a *DpnI* restriction digest was performed to digest the template DNA, leaving only the PCR-amplified plasmids intact. After briefly boiling the *DpnI* digest, the DNA plasmid was transformed into TOP10 competent cells and plated on LB / kanamycin agar plates overnight at 37°C. For each mutant, DNA plasmids were isolated from at least four independent colonies. A *BsgRI* restriction digest was then performed to verify the presence of the Pen-2 sequence within the plasmid DNA. The sequences of all mutant-containing plasmids were then verified by DNA sequencing.

### Example 5: Testing of mutant Pen-2 variants in cells

The functional activity of mutant Pen-2 variants (containing point mutations within the Pen-2 binding motif as described in Example 4) can be tested in cells that express low levels of endogenous levels of Pen-2. A suitable cell system in which the endogenous Pen-2 protein level is downregulated via RNA interference-mediated Pen-2 knockdown was described (Prokop et al., 2004. J. Biol. Chem. 279, 23255- 23261).
Such a Pen-2 knock-down cell line displays very little to no endogenous γ-secretase activity, as evidenced by the accumulation of α- and β-C-terminal fragments of APP in cell lysates (Prokop et al., 2004). γ-secretase activity can be reconstituted in such a cell line upon expression of recombinant exogenous wild-type Pen-2, but not by expression of mutant Pen-2, such as a C-terminal deletion mutant, that fails to be stably incorporated into γ-secretase protein complexes (Prokop et a]., 2004).
Testing of Pen-2 variants carrying point mutations in the Pen-2 binding motif (see example 4) is performed as follows:
Pen-2 mutants are transfected into Pen-2 knock-down cells and their ability to reconstitute γ-secretase activity is assessed as outlined by Prokop et al. (2004). Cells expressing such mutants that successfully reconstitute γ-secretase activity are subsequently tested for their sensitivity to both γ-secretase inhibitors and modulators. Since inhibitors target the enzyme complex' active site distinct from Pen-2, γ-secretase protein complexes containing Pen-2 variants carrying point mutations in the Pen-2 binding motif are expected to be sensitive to γ-secretase inhibitors, whereas they are expected to have lost their ability to bind γ-secretase modulators. Consequently, γ-secretase activity of cells expressing such a Pen-2 mutant is expected to be insensitive to γ-secretase modulators. Accordingly, cells expressing such a Pen-2 mutant can be utilized for identification and characterization of γ-secretase modulators.

### Example 6: Screening of compounds for gamma-secretase modulating activity in a cellular assay

Screening is carried out using SK-N-BE2 neuroblastoma cells carrying the APP 695 - wild type protein, grown in DMEM/NUT-mix F12 (HAM) provided by Invitrogen/Gibco (cat no. 31330-38) containing 5% Serum/Fe supplemented with 1% non-essential amino acids. Cells are grown to near confluency. The screening can be performed using the assay as described in Citron et al (1997) Nature Medicine 3: 67.

## Claims

1. A method for identifying a gamma-secretase modulator, comprising the following steps:
a) identifying a Pen-2 interacting compound, and
b) determining whether the Pen-2 interacting compound identified in step a) is capable of modulating gamma-secretase activity.

2. The method of claim 1, wherein in step a) a compound is identified which is capable of binding to the Pen-2 binding site **characterized by** SEQ ID NO:2.

3. The method of any of claims 1 or 2, wherein in step a) a given test compound is brought into contact with a peptide comprising the Pen-2 binding site as defined in claim 2 or a fragment or homologue thereof.

4. The method of claim 3, wherein it is determined whether the test compound is able to bind to the peptide comprising the Pen-2 binding site or a fragment or homologue thereof.

5. The method of any of claims 3 or 4, wherein the peptide comprising the Pen-2 binding site or the fragment or homologue thereof is a purified protein, part of a membrane preparation or expressed in a cell (on the surface or in a cell compartment).

6. The method of any of claims 3 to 5, wherein the peptide comprising the Pen-2 binding site is Pen-2.

7. The method of any of claims 4 to 6, wherein the ability to bind is tested by using a competitive binding assay, preferably by using a ligand, more preferably a gamma secretase modulator.

8. The method of any of claims 1 to 7, wherein said compound is selected from the group consisting of synthetic compounds, or organic synthetic drugs, more preferably small organic drugs, natural small molecule compounds, peptides or aptamers.

9. The method of any of claims 1 to 8, wherein in step b) the ability of gamma-secretase to cleave APP is measured.

10. The method of claim 9, wherein the ability of gamma secretase to produce Abeta 42 from APP is measured.

11. The method of any of claims 1 to 10, performed as a medium or high throughput screening.

12. A method for the identification of a gamma secretase inhibitor, comprising the steps of
a) providing a gamma-secretase protein complex comprising a mutated Pen-2 protein with a non functional Pen-2 binding site,
b) contacting the gamma-secretase protein complex as defined in step a) with a test compound, and
c) determining whether the test compound inhibits gamma-secretase activity.

13. The method of claim 12, wherein the proteins of the gamma-secretase protein complex defined in step a) have been expressed in a cell, preferably the same-cell.

14. The method of claim 13, wherein the cell is unable to express functional endogenous Pen-2.

15. The method of any of claims 12 to 14, wherein in step c) the ability of a test compound to inhibit APP cleavage is determined.

16. A method for preparing a pharmaceutical composition for the treatment of neurodegenerative diseases, comprising the following steps:
a) identifying a gamma-secretase modulator according to any of claims 1 to 11 or a gamma-secretase inhibitor according to any of claims 12 to 14 , and
b) formulating the gamma-secretase modulator or inhibitor to a pharmaceutical composition.

17. The method of claim 16, further comprising the step of mixing the identified compound with a pharmaceutically acceptable carrier.

18. A pharmaceutical composition comprising the gamma secretase modulator identified according to the method of any of claims 1 to 11 or the gamma-secretase inhibitor identified according to any of claims 12 to 14.

19. A pharmaceutical composition obtainable by the method according to any of claims 16 or 17.

20. The pharmaceutical composition according to any of claims 18 or 19 for the treatment of neurodegenerative disease such as Alzheimer's disease and related neurodegenerative disorders.

21. Use of a pharmaceutical composition according to any of claims 18 or 19 for the treatment of neurodegenerative disease such as Alzheimer's disease and related neurodegenerative disorders.

22. A method for treating or preventing a neurodegenerative disease, preferably Alzheimer's disease administering to a subject in need of such treatment or prevention a therapeutically effective amount of a pharmaceutical composition of any of claims 18 to 20.

23. The pharmaceutical composition according to any of claims 18 or 19 for the treatment of cancer, preferably colon cancer.

24. Use of a pharmaceutical composition according to any of claims 18 or 19 for the treatment of cancer, preferably colon cancer.

25. A method for treating or preventing cancer, preferably colon cancer administering to a subject in need of such treatment or prevention a therapeutically effective amount of a pharmaceutical composition of any of claims 18, 19 or 23.


### Annex to the application documents - subsequently filed sequences listing

SEQUENCE LISTING

<110>  CellZome AG

<120>  Methods for the identification of gamma-secretase modulators

<130>  CEL62545EP

<140>  06003238.0
<141>  2006-02-17

<160>  14

<170>  PatentIn version 3.3

<210>  1
<211>  101
<212>  PRT
<213>  Homo sapiens

<400>  1

Met Asn Leu Glu Arg Val Ser Asn Glu Glu Lys Leu Asn Leu Cys Arg
1               5                   10                  15

Lys Tyr Tyr Leu Gly Gly Phe Ala Phe Leu Pro Phe Leu Trp Leu Val
            20                  25                  30

Asn Ile Phe Trp Phe Phe Arg Glu Ala Phe Leu Val Pro Ala Tyr Thr
        35                  40                  45

Glu Gln Ser Gln Ile Lys Gly Tyr Val Trp Arg Ser Ala Val Gly Phe
    50                  55                  60

Leu Phe Trp Val Ile Val Leu Thr Ser Trp Ile Thr Ile Phe Gln Ile
65                  70                  75                  80

Tyr Arg Pro Arg Trp Gly Ala Leu Gly Asp Tyr Leu Ser Phe Thr Ile
                85                  90                  95

Pro Leu Gly Thr Pro
            100

<210>  2
<211>  59
<212>  PRT
<213>  Homo sapiens

<400>  2

Met Asn Leu Glu Arg Val Ser Asn Glu Glu Lys Leu Asn Leu Cys Arg
1               5                   10                  15

Lys Tyr Tyr Leu Gly Gly Phe Ala Phe Leu Pro Phe Leu Trp Leu Val
            20                  25                  30

Asn Ile Phe Trp Phe Phe Arg Glu Ala Phe Leu Val Pro Ala Tyr Thr
        35                  40                  45

Glu Gln Ser Gln Ile Lys Gly Tyr Val Trp Arg
    50                  55

<210>  3
<211>  294
<212>  PRT
<213>  Homo sapiens

<400>  3

Asp Glu Gln Leu Phe Gln Thr Ser Arg Leu Ile Leu Ile Gly Glu Thr
1               5                   10                  15

Ile Lys Ile Val Ile Glu Asp Tyr Val Gln His Leu Ser Gly Tyr His
            20                  25                  30

Phe Lys Leu Lys Phe Asp Pro Glu Leu Leu Phe Asn Lys Gln Phe Gln
        35                  40                  45

Tyr Gln Asn Arg Ile Ala Ala Glu Phe Asn Thr Leu Tyr His Trp His
    50                  55                  60

Pro Leu Leu Pro Asp Thr Phe Gln Ile His Asp Gln Lys Tyr Asn Tyr
65                  70                  75                  80

Gln Gln Phe Ile Tyr Asn Asn Ser Ile Leu Leu Glu His Gly Ile Thr
                85                  90                  95

Gln Phe Val Glu Ser Phe Thr Arg Gln Ile Ala Gly Arg Val Ala Gly
            100                 105                 110

Gly Arg Asn Val Pro Pro Ala Val Gln Lys Val Ser Gln Ala Ser Ile
        115                 120                 125

Asp Gln Ser Arg Gln Met Lys Tyr Gln Ser Phe Asn Glu Tyr Arg Lys
    130                 135                 140

Arg Phe Met Leu Lys Pro Tyr Glu Ser Phe Glu Glu Leu Thr Gly Glu
145                 150                 155                 160

Lys Glu Met Ser Ala Glu Leu Glu Ala Leu Tyr Gly Asp Ile Asp Ala
                165                 170                 175

Val Glu Leu Tyr Pro Ala Leu Leu Val Glu Lys Pro Arg Pro Asp Ala
            180                 185                 190

Ile Phe Gly Glu Thr Met Val Glu Val Gly Ala Pro Phe Ser Leu Lys
        195                 200                 205

Gly Leu Met Gly Asn Val Ile Cys Ser Pro Ala Tyr Trp Lys Pro Ser
    210                 215                 220

Thr Phe Gly Gly Glu Val Gly Phe Gln Ile Ile Asn Thr Ala Ser Ile
225                 230                 235                 240

Gln Ser Leu Ile Cys Asn Asn Val Lys Gly Cys Pro Phe Thr Ser Phe
                245                 250                 255

Ser Val Pro Asp Pro Glu Leu Ile Lys Thr Val Thr Ile Asn Ala Ser
            260                 265                 270

Ser Ser Arg Ser Gly Leu Asp Asp Ile Asn Pro Thr Val Leu Leu Lys
        275                 280                 285

Glu Arg Ser Thr Glu Leu
    290

<210>  4
<211>  294
<212>  PRT
<213>  Ovis ammon aries

<400>  4

Asp Glu Gln Leu Phe Gln Thr Ser Lys Leu Ile Leu Ile Gly Glu Thr
1               5                   10                  15

Ile Lys Ile Val Ile Glu Asp Tyr Val Gln His Leu Ser Gly Tyr His
            20                  25                  30

Phe Lys Leu Lys Phe Asp Pro Glu Leu Leu Phe Asn Gln Gln Phe Gln
        35                  40                  45

Tyr Gln Asn Arg Ile Ala Ser Glu Phe Asn Thr Leu Tyr His Trp His
    50                  55                  60

Pro Leu Leu Pro Asp Thr Phe Asn Ile Glu Asp Gln Glu Tyr Ser Phe
65                  70                  75                  80

Lys Gln Phe Leu Tyr Asn Asn Ser Ile Leu Leu Glu His Gly Leu Thr
                85                  90                  95

Gln Phe Val Glu Ser Phe Thr Arg Gln Ile Ala Gly Arg Val Ala Gly
            100                 105                 110

Gly Arg Asn Val Pro Ile Ala Val Gln Ala Val Ala Lys Ala Ser Ile
        115                 120                 125

Asp Gln Ser Arg Glu Met Lys Tyr Gln Ser Leu Asn Glu Tyr Arg Lys
    130                 135                 140

Arg Phe Ser Leu Lys Pro Tyr Thr Ser Phe Glu Glu Leu Thr Gly Glu
145                 150                 155                 160

Lys Glu Met Ala Ala Glu Leu Lys Ala Leu Tyr Ser Asp Ile Asp Val

                165                 170                 175

Met Glu Leu Tyr Pro Ala Leu Leu Val Glu Lys Pro Arg Pro Asp Ala
            180                 185                 190

Ile Phe Gly Glu Thr Met Val Glu Leu Gly Ala Pro Phe Ser Leu Lys
        195                 200                 205

Gly Leu Met Gly Asn Pro Ile Cys Ser Pro Gln Tyr Trp Lys Pro Ser
    210                 215                 220

Thr Phe Gly Gly Glu Val Gly Phe Lys Ile Ile Asn Thr Ala Ser Ile
225                 230                 235                 240

Gln Ser Leu Ile Cys Asn Asn Val Lys Gly Cys Pro Phe Thr Ser Phe
                245                 250                 255

Asn Val Gln Asp Pro Gln Pro Thr Lys Thr Ala Thr Ile Asn Ala Ser
            260                 265                 270

Ala Ser His Ser Arg Leu Asp Asp Ile Asn Pro Thr Val Leu Ile Lys
        275                 280                 285

Arg Arg Ser Thr Glu Leu
    290

<210> 5
<211> 276
<212> PRT
<213> Homo sapiens

<400> 5

Asp Glu Gln Leu Phe Gln Thr Thr Arg Leu Ile Leu Ile Gly Glu Thr
1               5                   10                  15

Ile Lys Ile Val Ile Glu Glu Tyr Val Gln Gln Leu Ser Gly Tyr Phe
            20                  25                  30

Leu Gln Leu Lys Phe Asp Pro Glu Leu Leu Phe Gly Val Gln Phe Gln
        35                  40                  45

Tyr Arg Asn Arg Ile Ala Met Glu Phe Asn His Leu Tyr His Trp His
    50                  55                  60

Pro Leu Met Pro Asp Ser Phe Lys Val Gly Ser Gln Glu Tyr Ser Tyr
65                  70                  75                  80

Glu Gln Phe Leu Phe Asn Thr Ser Met Leu Val Asp Tyr Gly Val Glu
                85                  90                  95

Ala Leu Val Asp Ala Phe Ser Arg Gln Ile Ala Gly Arg Ile Gly Gly
            100                 105                 110

Gly Arg Asn Met Asp His His Ile Leu His Val Ala Val Asp Val Ile
        115                 120                 125

Arg Glu Ser Arg Glu Met Arg Leu Gln Pro Phe Asn Glu Tyr Arg Lys
    130                 135                 140

Arg Phe Gly Met Lys Pro Tyr Thr Ser Phe Gln Glu Leu Val Gly Glu
145                 150                 155                 160

Lys Glu Met Ala Ala Glu Leu Glu Glu Leu Tyr Gly Asp Ile Asp Ala
                165                 170                 175

Leu Glu Phe Tyr Pro Gly Leu Leu Leu Glu Lys Cys His Pro Asn Ser
            180                 185                 190

Ile Phe Gly Glu Ser Met Ile Glu Ile Gly Ala Pro Phe Ser Leu Lys
        195                 200                 205

Gly Leu Leu Gly Asn Pro Ile Cys Ser Pro Glu Tyr Trp Lys Pro Ser
    210                 215                 220

Thr Phe Gly Gly Glu Val Gly Phe Asn Ile Val Lys Thr Ala Thr Leu
225                 230                 235                 240

Lys Lys Leu Val Cys Leu Asn Thr Lys Thr Cys Pro Tyr Val Ser Phe
                245                 250                 255

Arg Val Pro Asp Ala Ser Gln Asp Asp Gly Pro Ala Val Glu Arg Pro
            260                 265                 270

Ser Thr Glu Leu
        275

<210>  6
<211>  21
<212>  DNA
<213>  Artificial Sequence

<220>
<223>  Primer in vitro mutagenesis F23A top

<400>  6
ctgggggggg ctgctttcct g                                                21

<210>  7
<211>  21
<212>  DNA
<213>  Artificial Sequence

<220>
<223>  Primer in vitro mutagenesis F23A bottom

<400>  7
caggaaagca gcccccccca g                                                21

<210>  8
<211>  24
<212>  DNA
<213>  Artificial Sequence

<220>
<223>  Primer in vitro mutagenesis Y56A top

<400>  8
caaatcaaag gcgctgtctg gcgc                                              24


<210>  9
<211>  24
<212>  DNA
<213>  Artificial Sequence

<220>
<223>  Primer in vitro mutagenesis Y56A bottom

<400>  9
gcgccagaca gcgcctttga tttg                                              24


<210>  10
<211>  22
<212>  DNA
<213>  Artificial Sequence

<220>
<223>  Primer in vitro mutagenesis W58A top

<400>  10
ggctatgtcg cgcgctcagc tg                                                22


<210>  11
<211>  22
<212>  DNA
<213>  Artificial Sequence

<220>
<223>  Primer in vitro mutagenesis W58A bottom

<400>  11
cagctgagcg cgcgacatag cc                                                22


<210>  12
<211>  22
<212>  DNA
<213>  Artificial Sequence

<220>
<223>  Primer in vitro mutagenesis R59A top

<400>  12
ctatgtctgg gcctcagctg tg                                                22


<210>  13
<211>  22
<212>  DNA
<213>  Artificial Sequence

<220>

<223>  Primer in vitro mutagenesis R59A bottom

<400>  13
cacagctgag gcccagacat ag                                                22


<210>  14
<211>  101
<212>  PRT
<213>  Homo Sapiens


<220>
<221>  223
<222>  (1)..(101)
<223>  Peptide matching to Pen-2 identified in a drug pulldown
       experiment using the SK-N-BE2 neuroblastoma cell line

<400>  14

Met Asn Leu Glu Arg Val Ser Asn Glu Glu Lys Leu Asn Leu Cys Arg
1               5                   10                  15

Lys Tyr Tyr Leu Gly Gly Phe Ala Phe Leu Pro Phe Leu Trp Leu Val
            20                  25                  30

Asn Ile Phe Trp Phe Phe Arg Glu Ala Phe Leu Val Pro Ala Tyr Thr
        35                  40                  45

Glu Gln Ser Gln Ile Lys Gly Tyr Val Trp Arg Ser Ala Val Gly Phe
    50                  55                  60

Leu Phe Trp Val Ile Val Leu Thr Ser Trp Ile Thr Ile Phe Gln Ile
65                  70                  75                  80

Tyr Arg Pro Arg Trp Gly Ala Leu Gly Asp Tyr Leu Ser Phe Thr Ile
                85                  90                  95

Pro Leu Gly Thr Pro
            100